# EUROPÄISCHE PATENTSCHRIFT

(11) **EP 3 624 884 B1**
(45) Veröffentlichungstag und Bekanntmachung des Hinweises auf die Patenterteilung: **14.04.2021**
(21) Anmeldenummer: 18725211.9
(22) Anmeldetag: 16.05.2018
(51) Int. Cl.: A61M 16/20, A61M 16/08, F16K 1/52, F16K 7/17

(54) **EXSPIRATIONSVENTILANORDNUNG FÜR EINE BEATMUNGSVORRICHTUNG MIT VORRICHTUNG ZUR AUFNAHME EINES DRUCKSENSORS**
EXHALATION VALVE ARRANGEMENT FOR A VENTILATOR APPARATUS WITH AN APPARATUS FOR RECEIVING A PRESSURE SENSOR
ENSEMBLE CLAPET D'EXPIRATION POUR UN DISPOSITIF RESPIRATOIRE, DOTÉ D'UN DISPOSITIF POUR RECEVOIR UN CAPTEUR DE PRESSION

(30) Priorität: 17.05.2017 DE 102017208349
(43) Veröffentlichungstag der Anmeldung: 25.03.2020
(73) Patentinhaber: Hamilton Medical AG, 7402 Bonaduz (CH)
(72) Erfinder: FRANKE, Karolin, 8003 Zurich (CH); BREITRUCK, Felix, 9470 Buchs (CH)
(74) Vertreter: Ruttensperger Lachnit Trossin Gomoll
(86) Internationale Anmeldenummer: PCT/EP2018/062795
(87) Internationale Veröffentlichungsnummer: WO 2018/210958

(56) Entgegenhaltungen:
- EP-A2- 1 491 227
- EP-B1- 2 663 354
- WO-A1-2008/123831
- CN-A- 101 642 597
- CN-U- 203 417 393
- CN-U- 203 943 996
- GB-A- 1 551 226
- US-A- 5 127 400
- US-A1- 2014 305 431
- US-B1- 7 207 332

## Beschreibung

Die vorliegende Erfindung betrifft eine Exspirationsventilanordnung für eine Exspirationsleitung einer Beatmungsvorrichtung zur künstlichen Beatmung von Patienten, wobei die Exspirationsventilanordnung in einer Exspirationsströmungsrichtung durchströmbar ist und umfasst:
- einen stromaufwärtigen Atemgaskanal, welcher sich längs einer ersten Kanalbahn erstreckt und welcher mit einem vom Patienten kommenden Abschnitt der Exspirationsleitung verbunden oder verbindbar ist,
- einen stromabwärtigen Atemgaskanal, welcher sich längs einer zweiten Kanalbahn erstreckt und welcher mit einer Atemgas-Senke, wie etwa der Außenumgebung, verbunden oder verbindbar ist,
- eine einen Ventilkörper und einen Ventilsitz aufweisende Ventilbaugruppe, welche zwischen dem stromaufwärtigen und dem stromabwärtigen Atemgaskanal derart vorgesehen ist, dass sie bei einem vorbestimmten ersten Atemgasüberdruck im stromaufwärtigen Atemgaskanal relativ zum stromabwärtigen Atemgaskanal eine exspiratorische Atemgasströmung vom stromaufwärtigen Atemgaskanal in den stromabwärtigen Atemgaskanal zulässt und dass sie bei einem vorbestimmten zweiten Atemgasüberdruck im stromabwärtigen Atemgaskanal relativ zum stromaufwärtigen Atemgaskanal eine Gasströmung vom stromabwärtigen Atemgaskanal in den stromaufwärtigen Atemgaskanal sperrt.

Exspirationsventilanordnungen, wie die oben Beschriebene, werden in Beatmungsvorrichtungen zur wenigstens unterstützenden künstlichen Beatmung von Patienten verwendet, um einen den natürlichen Atmungszyklen richtungsmäßig entsprechenden Atemgasfluss zu erzeugen. Die Beatmungsvorrichtungen weisen üblicherweise eine Inspirationsleitung und eine Exspirationsleitung auf mit einer in der Inspirationsleitung vorgesehenen Inspirationsventilanordnung und mit einer in der Exspirationsleitung angeordneten Exspirationsventilanordnung. Die Inspirationsventilanordnung lässt bei makroskopischer Betrachtung der Vorgänge im Wesentlichen nur einen inspiratorischen Atemgasfluss zum Patienten hin zu. Die Exspirationsventilanordnung lässt bei ebenso makroskopischer Betrachtung im Wesentlichen nur einen exspiratorischen Atemgasfluss in Exspirationsströmungsrichtung vom Patienten weg zu.

Bei stärker detaillierter Betrachtung der Exspirationsventilanordnung können dort über das bloße Öffnen und Schließen hinaus weitere für einen Beatmungsvorgang wichtige Vorgänge stattfinden, wie etwa ein Aufrechterhalten einer residualen Durchströmungsöffnung an der Ventilbaugruppe gegen Ende eines Exspirationsvorgangs, um sicherzustellen, dass in der Exspirationsleitung und damit auch in der mit dieser strömungstechnisch kommunizierenden Patientenlunge ein positiver endexspiratorischer Druck (PEEP = "positive end exspiratory pressure") aufrechterhalten wird.

Die eingangs genannten vorbestimmten Atemgasüberdrucke: erster und der zweiter Atemgasüberdruck, müssen weder betragsmäßig übereinstimmen, noch auf demselben Atemgas-Druckniveau liegen.

Da der stromabwärtige Atemgaskanal bei den meisten bekannten Exspirationsventilanordnungen zur Außenumgebung als einer Atemgas-Senke der die Exspirationsventilanordnung tragenden Beatmungsvorrichtung hin öffnet, wird dem Atemgas im stromabwärtigen Atemgaskanal üblicherweise von seinem zur Außenumgebung hin öffnenden Längsende her der Umgebungsdruck aufgezwungen. Daher ist der erste Atemgasüberdruck in der Regel ein Überdruck im stromaufwärtigen Atemgaskanal bezüglich des Drucks der Außenumgebung, wie es für einen Exspirationsvorgang charakteristisch ist, und ist der zweite Atemgasüberdruck ein Überdruck des Umgebungsdrucks bezüglich eines im stromaufwärtigen Atemgaskanal herrschenden geringeren Drucks, wie es beispielsweise für einen Inspirationsvorgang charakteristisch ist.

Die Aussage, dass die Ventilbaugruppe bei Vorliegen des ersten Atemgasüberdrucks eine Exspirationsströmung in Exspirationsströmungsrichtung vom stromaufwärtigen Atemgaskanal zum stromabwärtigen Atemgaskanal gestattet und bei Vorliegen des zweiten vorbestimmten Atemgasüberdrucks eine Strömung in entgegengesetzte Richtung verhindert, soll nicht ausschließen, dass bei von dem ersten und dem zweiten vorbestimmten Atemgasüberdruck abweichenden Druckverhältnissen Betriebszustände der Ventilbaugruppe vorliegen, die eingangs der Anmeldung nicht genannt sind. Entscheidend ist nur, dass bei Vorliegen der genannten Atemgasüberdrucke die genannten Betriebszustände der Ventilbaugruppe vorliegen.

Der erste und der zweite vorbestimmte Atemgasüberdruck können jeweils ein Atemgasüberdruck-Wertebereich sein, um unterschiedliche Patienten und Patiententypen sicher beatmen zu können.

Die Begriffe "stromaufwärtig" und "stromabwärtig" beziehen sich an der Exspirationsventilanordnung jeweils auf die konstruktiv eindeutig an dieser erkennbare Exspirationsströmungsrichtung, die bei Vorliegen des vorbestimmten ersten Atemgasüberdrucks durch die Exspirationsventilanordnung hindurch ermöglicht wird.

Eine gattungsgemäße Exspirationsventilanordnung ist aus der WO 02/076544 A1 bekannt.

Für eine möglichst exakte Funktionssteuerung der Exspirationsventilanordnung, insbesondere um den von der Exspirationsventilanordnung bewirkten PEEP am Ende eines Exspirationsvorgangs möglichst exakt einstellen zu können, ist die Kenntnis des stromaufwärts der Ventilbaugruppe herrschenden Atemgasdrucks hilfreich.

In einer Exspirationsleitung einer Beatmungsvorrichtung ist häufig an deren proximalem Ende ein nach dem Differenzdruck-Prinzip arbeitender Flusssensor angeordnet, über welchen aufgrund seines Funktionsprinzips auch Information über den Druck des Atemgases in der Exspirationsleitung verfügbar ist. Allerdings liegen zwischen der Messstelle des Atemgasdrucks am proximalen Ende der Exspirationsleitung und einer nahe bei der Ventilbaugruppe, jedoch stromaufwärts derselben gelegenen Messstelle konstruktive Fehlereinflussmöglichkeiten, etwa bedingt durch die Elastizität eines die Exspirationsleitung bis zur Exspirationsventilanordnung bildenden Exspirationsschlauchs, so dass die Druckmessung am proximalem Ende der Exspirationsleitung wenig aussagekräftig für den tatsächlich im distalen Endbereich der Exspirationsleitung im stromaufwärtigen Atemgaskanal stromaufwärts der Ventilbaugruppe herrschenden Atemgasdruck ist.

Es ist daher Aufgabe der vorliegenden Erfindung, die eingangs genannte Exspirationsventilanordnung derart zu verbessern, dass sie eine Druckerfassung im exspiratorischen Atemgas stromaufwärts der Ventilbaugruppe in geringem Abstand zu dieser ermöglicht. Dies ist in der Regel konstruktiv deshalb schwierig, weil der stromaufwärtige Atemgaskanal der Exspirationsventilanordnung häufig bauartbedingt nicht unmittelbar zugänglich ist. Denn er ist in unmittelbarer Annäherung an die Ventilbaugruppe in Exspirationsströmungsrichtung in der Regel radial außen von einer Ringkammer umgeben.

Die US 5127400 A offenbart ein Exspirationsventil für ein Beatmungsgerät mit einem Ventilkörper, aufweisend einen Einlassstutzen, einen Auslassstutzen, einen Ventilsitz und einen Druckmessstutzen, wobei sich der Ventilsitz zwischen dem Einlassstutzen und dem Auslassstutzen befindet, und wobei sich der Druckmessstutzen zwischen dem Ventilsitz und dem Einlassstutzen befindet. Bei diesem Exspirationsventil bewirkt ein Zusammenwirken von Ventilsitz und Membran ("diaphragm") während der Exspiration eine variable Durchflussfläche.

Jede der EP 1491227 A2, EP 2663354 B1, CN 203943996 U, CN 101642597 A, CN 203417393 U und US 7207332 B1 offenbart ein Exspirationsventil für ein Beatmungsgerät, mit einem Druckmessstutzen.

Erfindungsgemäß wird die genannte Aufgabe gelöst durch eine Exspirationsventilanordnung nach Anspruch 1 und durch eine Beatmungsvorrichtung nach Anspruch 15. Die Unteransprüche 2 bis 14 definieren bevorzugte Ausführungsbeispiele der Erfindung. Eine erfindungsgemäße Exspirationsventilanordnung weist eine Bypass-Kammer auf, die strömungsmechanisch mit dem stromaufwärtigen Atemgaskanal kommuniziert und sich ausgehend vom stromaufwärtigen Atemgaskanal in den Bereich des stromabwärtigen Atemgaskanals erstreckt und dort zur Ankopplung eines Gasdrucksensors ausgebildet ist.

Da der stromabwärtige Atemgaskanal üblicherweise zur Außenumgebung der Exspirationsventilanordnung oder der Beatmungsvorrichtung insgesamt hin öffnet, ist der stromabwärtige Atemgaskanal in vielen Fällen einfacher zugänglich als der stromaufwärtige Atemgaskanal. Die sich in den Bereich des stromabwärtigen Atemgaskanals erstreckende Bypass-Kammer ist daher ebenfalls einfach zugänglich, und zwar in der Regel auch im bereits in eine Beatmungsvorrichtung eingebauten Zustand. Die Bypass-Kammer schafft somit einen messtechnischen Zugang zu dem verhältnismäßig stark verborgenen oder verbauten stromaufwärtigen Atemgaskanal und macht den dort herrschenden Atemgasdruck für einen an die Bypass-Kammer angekoppelbaren Gasdrucksensor erfassbar. Für die Ankopplung des Gasdrucksensors ist die Bypass-Kammer eigens ausgebildet.

Bevorzugt ist die Bypass-Kammer an einem Verbindungsbereich derselben, etwa an ihrem einen Längsende, mit dem stromaufwärtigen Atemgaskanal verbunden und öffnet beispielsweise in diesen und ist an einem Ankopplungsbereich, etwa an ihrem entgegengesetzten Längsendbereich, zur Ankopplung des Gasdrucksensors ausgebildet. Die Ausbildung der Bypass-Kammer zur Ankopplung eines Drucksensors kann in einem einfachen, aber zuverlässig wirkenden Ausführungsbeispiel dadurch realisiert sein, dass ein Sensor-Ankopplungswandabschnitt einer die Bypass-Kammer begrenzenden Wandung eine Sensor-Ankopplungsöffnung aufweist, welche den Sensor-Ankopplungswandabschnitt der Bypass-Kammer durchsetzt. Durch die Sensor-Ankopplungsöffnung ist somit das Innere der Bypass-Kammer von außen grundsätzlich zugänglich.

In vorteilhafter Weise kann dabei dem jeweiligen Benutzer, etwa Pflegepersonal oder einem verantwortlichen Arzt, überlassen werden, ob eine Erfassung des Atemgasdrucks im stromaufwärtigen Atemgaskanal nahe der Ventilbaugruppe überhaupt erforderlich oder wenigstens gewünscht ist oder nicht, wenn die Sensor-Ankopplungsöffnung durch einen Verschluss verschlossen ist, welcher wahlweise geöffnet werden kann, um einen Gasdrucksensor messtechnisch an die Bypass-Kammer anzukoppeln. Es existieren nämlich auch einfachere Beatmungsvorrichtungen, bei welchen die Erfassung des Drucks des stromaufwärtigen Atemgases nicht vorgesehen ist und welche zu der signaltechnischen Verarbeitung eines solchen Druckerfassungssignals nicht ausgerüstet sind. Auch in solchen Beatmungsvorrichtungen kann die erfindungsgemäße Exspirationsventilanordnung verwendet werden, indem man dann die Sensor-Ankopplungsöffnung einfach durch den Verschluss verschlossen belässt.

Grundsätzlich kann vorgesehen sein, dass der Verschluss zur Sicherung vor einem unbeabsichtigten Öffnen und damit vor einer Fehlfunktion der Beatmungsvorrichtung eine verhältnismäßig komplizierte Öffnungsbewegung erfordert, um die Sensor-Ankopplungsöffnung zu öffnen und für die messtechnische Ankopplung des Gasdrucksensors zugänglich zu machen. Beispielsweise kann der Verschluss von der Sensor-Ankopplungsöffnung abschraubbar sein, wobei zur Sicherheit vor einem unbeabsichtigten Öffnen der Sensor-Ankopplungsöffnung vorzugsweise mehrere Umdrehungen des Verschlusses notwendig sind, bevor dieser aus einer Verschlussstellung so weit gelöst ist, dass er von der Sensor-Ankopplungsöffnung abgehoben werden kann.

Häufig kommt es jedoch beim Einsatz von medizinischen lebenserhaltenden Geräten stark auf den Faktor Zeit an, so dass die Exspirationsventilanordnung möglichst schnell an einer Beatmungsvorrichtung einsetzbar sein soll. Daher ist gemäß einer bevorzugten Weiterbildung der vorliegenden Erfindung vorgesehen, dass der Verschluss eine durchstechbare Verschluss-Membran umfasst, welche aus einem im Vergleich zum Material des umgebenden Sensor-Ankopplungswandabschnitts weicheren Material gebildet ist. Ein solches weicheres Material kann ein Elastomer sein, insbesondere ein thermoplastisches Elastomer (TPE). Bewährt haben sich dabei vor allem Natur- oder Silikonkautschuk. Ein die Verschluss-Membran einfassender Randbereich des Verschlusses kann dann mit einem die Sensor-Ankopplungsöffnung einfassenden Grenzbereich des Sensor-Ankopplungswandabschnitts beispielsweise verklebt, durch Vulkanisation, durch 2-Komponenten-Spritzguss oder/und formschlüssig verbunden sein.

Alternativ oder zusätzlich kann der Verschluss mit einem im betriebsbereiten Zustand der Exspirationsventilanordnung mit dem Sensor-Ankopplungswandabschnitt zusammenwirkenden Zusatzbauteil verbunden sein. Ein solches Zusatzbauteil kann ein Stopfen sein, welcher beispielsweise eine bei der Herstellung der Bypass-Kammer unvermeidlich entstehende Öffnung verschließt, die nicht die Sensor-Ankopplungsöffnung ist. Das Zusatzbauteil kann eine Ergänzungsöffnung aufweisen und derart in oder an der Exspirationsventilanordnung vorgesehen sein, dass sich die Sensor-Ankopplungsöffnung und die Ergänzungsöffnung wenigstens überlagern. Vorzugsweise fluchten die beiden Öffnungen miteinander, zur Bereitstellung ausreichender Ankopplungsfläche für die messtechnische Ankopplung des Gasdrucksensors. Der Verschluss kann auch hier eine Verschluss-Membran umfassen, deren sie einfassender Randbereich abweichend vom oben Gesagten mit einem die Ergänzungsöffnung einfassenden Einfassungsbereich des Zusatzbauteils verbunden sein kann, wiederum unter Ausnutzung der oben bereits genannten Wirkprinzipien. Das Zusatzbauteil kann beispielsweise aus Polybutylenterephthalat oder Polyethylenterephthalat hergestellt sein.

Ein den stromabwärtigen Atemgaskanal bildendes Bauteil, insbesondere das unten genannte Kanalbauteil, kann aus Polyester, insbesondere Copolyester, oder Polycarbonat gebildet sein. Andere Werkstoffe sind nicht ausgeschlossen.

Der Vorteil einer derartigen weichelastischen Membran liegt nicht nur darin, dass sie schnell durchstochen und damit die Exspirationsventilanordnung schnell einsatzbereit gemacht werden kann. Ein weiterer Vorteil liegt auch darin, dass die so gebildete weichelastische Membran ähnlich einem Septum das sie durchstechende Bauteil abdichtend umschließen kann, so dass nach einem Durchstechen der Verschluss-Membran eine Gas-Fehlströmung zwischen der Bypass-Kammer und damit dem stromaufwärtigen Atemgaskanal einerseits und einer äußeren Umgebung der Bypass-Kammer ausgeschlossen ist. Bevorzugt wird die Verschluss-Membran durch ein Leitungsrohr durchstochen, welches die Bypass-Kammer messtechnisch mit einem Drucksensor verbindet.

"Messtechnisch verbinden" bzw. "messtechnisch ankoppeln" bedeutet im Sinne der vorliegenden Anmeldung, dass der so angekoppelte Gasdrucksensor durch die Verbindung bzw. Ankopplung in der Lage ist, den Druck in der Bypass-Kammer zu erfassen. Da die Bypass-Kammer wiederum mit dem stromaufwärtigen Atemgaskanal strömungstechnisch kommuniziert, kann so der angekoppelte Gasdrucksensor den Druck des Atemgases im stromaufwärtigen Atemgaskanal erfassen.

Weiterhin ist es für die Betriebssicherheit der Exspirationsventilanordnung vorteilhaft, wenn das Risiko von sich lösenden Bauteilen und Bauteilabschnitten ausgeschlossen werden kann. Somit können keine von der Exspirationsventilanordnung stammenden Bauteilabschnitte zum Patienten gelangen und von diesem versehentlich eingeatmet werden. Wenngleich dieses Risiko in einer Exspirationsleitung ohnehin faktisch nicht besteht, kann das Risiko eines Abreißens oder Loslösens von Membranbestandteilen beim Durchstechen der Verschluss-Membran dadurch gering gehalten oder sogar vollständig ausgeschlossen werden, dass die Verschluss-Membran einen Normalbereich und einen vom Normalbereich verschiedenen Materialschwächungsbereich aufweist, in welchem die Reißfestigkeit der Membran, verglichen mit dem Normalbereich, vermindert ist.

So kann gewährleistet werden, dass die Verschluss-Membran beim Durchstechen definiert aufreißt. Nicht nur ist durch ein definiertes Aufreißen bzw. durch eine definierte Zerstörung der Verschluss-Membran die Dichtungswirkung der Membran nach dem Durchstechen gewährleistet, da die nach dem Durchstechen vorliegende Membrangestalt der Verschluss-Membran bekannt und vorhersagbar ist. Außerdem ermöglicht die so mit einer Soll-Reißstelle bzw. einem Soll-Reißbereich versehene Verschluss-Membran die Verwendung von Ankopplungsrohren mit verhältnismäßig großem Durchmesser, was auf die Genauigkeit des Messergebnisses von mit derart großen Ankopplungsrohren oder -schläuchen angekoppelten Gasdrucksensoren einen positiven Einfluss hat. Ohne den Materialschwächungsbereich ist das Aufreißverhalten der Membran umso weniger gut vorhersagbar, je größer das die Membran durchstechende Objekt ist.

Der Materialschwächungsbereich kann in einfacher Weise dadurch gebildet sein, dass die Verschluss-Membran im Materialschwächungsbereich eine geringere Materialdicke aufweist als im Normalbereich.

Die geringere Materialdicke kann durch Einschnitte oder/und durch Einprägungen in der Verschluss-Membran bewirkt sein. Die Einschnitte gehen selbstverständlich nicht vollständig in Dickenrichtung durch das Material der Verschluss-Membran hindurch, sondern sind lediglich Teileinschnitte über einen Teil der Dickenerstreckung der Verschluss-Membran.

Um ein definiertes und auch regelmäßiges Aufreißverhalten der Verschluss-Membran bewirken zu können, bei welcher es auf eine Orientierung des die Verschluss-Membran durchstechenden Objekts, etwa einen Ankopplungsschlauch oder ein Ankopplungsrohr, nicht ankommt, ist bevorzugt der Materialschwächungsbereich musterartig in der Verschluss-Membran ausgebildet.

Beispielsweise kann der Materialschwächungsbereich einen Ring oder mehrere konzentrische Ringe aufweisen oder/und der Materialschwächungsbereich kann wenigstens zwei sich in der Membranmitte schneidende, die Verschluss-Membran jeweils in möglichst gleiche Membran-Flächenteile unterteilende Linien umfassen. Im bevorzugten Fall einer kreisförmigen Verschluss-Membran verlaufen diese Linien längs von Durchmessern.

Eine einfache und Bauraum sparende Ausgestaltung der Exspirationsventilanordnung, welche trotz der Ausbildung der Bypass-Kammer in bereits bestehende Beatmungsvorrichtungen einsetzbar ist, kann dadurch erhalten werden, dass ein Abschnitt einer den stromabwärtigen Atemgaskanal einfassenden Wandung den Sensor-Ankopplungswandabschnitt der Bypass-Kammer bildet. Dann kann nämlich die Bypass-Kammer im stromabwärtigen Atemgaskanal verlaufen, so dass der stromabwärtige Atemgaskanal eine Außenabmessung und Außengestalt aufweisen kann, wie sie eine baugleiche Exspirationsventilanordnung ohne Bypass-Kammer hat.

Somit kann die Exspirationsventilanordnung in bereits bekannter Gestalt und mit bereits bekannten Abmessungen hergestellt werden. Dies ist auch dadurch möglich, da für die Bypass-Kammer kein großes Kammervolumen benötigt wird und der stromabwärtige Atemgaskanal üblicherweise ohnehin einen großen Strömungsquerschnitt aufweist, welcher um einen Querschnitt der Bypass-Kammer verringert werden kann, ohne dass dies die Funktion des stromabwärtigen Atemgaskanals und damit der Exspirationsventilanordnung nennenswert stört.

Im Bereich des Sensor-Ankopplungswandabschnitt ist der stromabwärtige Atemgaskanal mit der in ihm liegenden Bypass-Kammer, wie oben bereits ausgesagt, zur Gewichtseinsparung nicht doppelwandig, sondern nur mit einer Einfachwandung ausgebildet. Da die Bypass-Kammer bei Betrachtung einer zur zweiten Kanalbahn orthogonalen Schnittebene eine geringere Querschnittsfläche aufweist, verläuft jedoch ein Teil einer die Bypass-Kammer nach radial außen begrenzenden Trennwand mit Abstand von einer den stromabwärtigen Atemgaskanal nach radial außen begrenzenden Kanalwandung.

Zur Verringerung des Risikos von sich in der Exspirationsventilanordnung ablösenden oder trennenden Bauteilabschnitten, welche möglicherweise lose in den Atemgaskanälen unvorhersehbare Wege zurücklegen können, ist bevorzugt die Exspirationsventilanordnung möglichst umfänglich einstückig hergestellt. Mit Bezug auf die für die vorliegende Erfindung wichtige Bypass-Kammer bedeutet dies bevorzugt, dass eine im stromabwärtigen Atemgaskanal die Bypass-Kammer vom stromabwärtigen Atemgaskanal trennende Trennwand einstückig mit der den stromabwärtigen Atemgaskanal einfassenden Kanalwandung ausgebildet ist.

Zur weiteren Gewährleistung eines möglichst hohen Bauteilzusammenhalts ist es vorteilhaft, etwaige Fügestellen in der Exspirationsventilanordnung möglichst zu reduzieren. Daher kann gemäß einer Weiterbildung vorgesehen sein, dass ein den stromaufwärtigen Atemgaskanal einfassendes erstes Exspirationsrohr, ein den stromabwärtigen Atemgaskanal einfassendes zweites Exspirationsrohr und die die Bypass-Kammer vom stromabwärtigen Atemgaskanal trennende Trennwand einstückig als Kanalbauteil ausgebildet sind.

Ein solches Kanalbauteil kann beispielsweise im Spritzgussverfahren einstückig hergestellt werden, etwa durch Verwendung von Kernen und Schiebern. Bevorzugt erstrecken sich sowohl der stromaufwärtige wie auch der stromabwärtige Atemgaskanal jeweils längs einer geradlinigen Kanalachse als der ersten bzw. der zweiten Kanalbahn. Bevorzugt schließen die erste und die zweite Kanalachse miteinander einen Winkel ein. Ebenso bevorzugt schneiden die verlängert gedachten Kanalachsen einander. Dies ermöglicht nicht nur eine kompakte räumliche Bauweise des Kanalbauteils, sondern ermöglicht auch, den Ventilkörper als einen ein Längsende des stromaufwärtigen Atemgaskanals überspannenden Membran-Ventilkörper auszubilden und ermöglicht, den Ventilsitz an dem Längsende des stromaufwärtigen Atemgaskanals auszubilden.

Der stromaufwärtige Atemgaskanal kann zumindest in seinem sich der Ventilbaugruppe in Exspirationsströmungsrichtung annähernden Längsende radial außen von einer Ringkammer umgeben sein, von welcher der stromabwärtige Atemgaskanal abzweigt. Die Ringkammer liegt dann strömungstechnisch in Exspirationsströmungsrichtung zwischen dem stromaufwärtigen und dem stromabwärtigen Atemgaskanal. Der Membran-Ventilkörper kann an einer die Ringkammer einfassenden Wandung festgelegt sein.

Für eine einfache, aber dennoch zuverlässige Sichtkontrolle der Exspirationsventilanordnung ist es außerdem vorteilhaft, wenn der Membran-Ventilkörper und die Verschluss-Membran mit zueinander parallelen Membranflächen an der Exspirationsventilanordnung vorgesehen sind.

In der Regel wird der Membran-Ventilkörper derart in eine Beatmungsvorrichtung eingebaut, dass der Membran-Ventilkörper in der betriebsbereiten Stellung der Exspirationsventilanordnung in der Beatmungsvorrichtung durch die Schwerkraft zum Ventilsitz hin, also zu einer Schließstellung hin, vorbelastet ist. Außerdem befindet sich häufig auf der von den Atemgaskanälen abgewandten Seite des Membran-Ventilkörpers eine Einrichtung der Beatmungsvorrichtung, welche dazu ausgebildet ist, den Membran-Ventilkörper mit einer vorbestimmten oder einstellbaren in Schließrichtung wirkenden Kraft zu beaufschlagen. Dies kann eine mechanische Einrichtung sein, etwa ein Stößel und dergleichen oder dies kann eine pneumatische Einrichtung sein, etwa ein mit Druck beaufschlagbarer Gasbehälter, von welchem der Membran-Ventilkörper einen Teil der Begrenzungsfläche bildet.

Daher wird häufig die Exspirationsventilanordnung entgegen der Schwerkraftwirkungsrichtung in das Gehäuse einer Beatmungsvorrichtung in seine betriebsbereite Stellung eingeführt.

In vorteilhafter Weise kann in dem Gehäuse der Beatmungsvorrichtung ein Ankopplungsrohr zur Ankopplung eines Gasdrucksensors bereits fest angeordnet sein. Der Gasdrucksensor kann in vorteilhaft einfacher Weise bereits mit dem Einbau der Exspirationsventilanordnung in die Beatmungsvorrichtung an den stromaufwärtigen Atemgaskanal angekoppelt werden, wenn die Verschluss-Membran bei betriebsbereit angeordneter Exspirationsventilanordnung orthogonal zur Schwerkraftwirkungsrichtung angeordnet ist, wobei ihre von der Bypass-Kammer wegweisende Außenseite entgegen der Schwerkraftwirkungsrichtung weist. Bei Einführung der Exspirationsventilanordnung in ein Gehäuse der Beatmungsvorrichtung kann so die Verschluss-Membran vom gehäusefesten Ankopplungsrohr im Zuge der Einführbewegung durchstoßen werden.

Die vorliegende Erfindung betrifft ebenso eine Beatmungsvorrichtung zur künstlichen Beatmung von Patienten mit einer Atemgas-Liefervorrichtung, von welcher eine Inspirationsleitung zu einer Patienten-Beatmungsschnittstelle führt, von welcher wiederum eine Exspirationsleitung zu einer Atemgas-Senke, wie etwa der Umgebungsatmosphäre, führt. Erfindungsgemäß ist zur Erzielung der oben genannten Wirkungen und Vorteile vorgesehen, dass in der Exspirationsleitung eine Exspirationsventilanordnung vorgesehen ist, wie sie oben Beschrieben und weitergebildet ist. Dabei ist der stromaufwärtige Atemgaskanal durch einen Abschnitt der Exspirationsleitung mit der Patienten-Beatmungsschnittstelle zur Übertragung von exspiratorischem Atemgas von der Patienten-Beatmungsschnittstelle verbunden.

Die oben bei der Erläuterung der erfindungsgemäßen Exspirationsventilanordnung dargelegten Ausbildungen einer Beatmungsvorrichtung sind erfindungsgemäße Weiterbildungen der im vorhergehenden Absatz genannten Beatmungsvorrichtung.

Die vorliegende Erfindung wird nachfolgend anhand der beiliegenden Zeichnungen näher erläutert werden. Es stellt dar:
- Figur 1: eine Beatmungsvorrichtung mit einer erfindungsgemäßen Ausführungsform einer Exspirationsventilanordnung,
- Figur 2: eine perspektivische Ansicht einer ersten erfindungsgemäßen Ausführungs-form einer Exspirationsventilanordnung der vorliegenden Anmeldung,
- Figur 3: eine Draufsicht eines Verschlusses einer Sensor-Ankopplungsöffnung in Gestalt einer Verschluss-Membran an der ersten Ausführungsform der Exspirationsventilanordnung,
- Figur 4: eine Längsschnittansicht durch die Exspirationsventilanordnung von Figur 2 in einer die erste und die zweite Kanalbahn enthaltenden Schnittebene,
- Figur 5: eine Detailansicht des Längsschnitts von Figur 4 mit intakter Verschluss-Membran und mit Abstand davon außerhalb der Bypass-Kammer angeordnetem Ankopplungsrohr,
- Figur 6: die Ansicht von Figur 5 mit die Sensor-Ankopplungsöffnung nach einem Durchstoßen der Verschluss-Membran durchsetzenden Ankopplungsrohr,
- Figur 7: eine der Ansicht von Figur 2 entsprechende Längsschnittansicht durch eine zweite Ausführungsform einer Exspirationsventilanordnung der vorliegen-den Anmeldung und
- Figur 8: eine Detailansicht des Längsschnitts von Figur 7 mit intakter Verschluss-Membran.

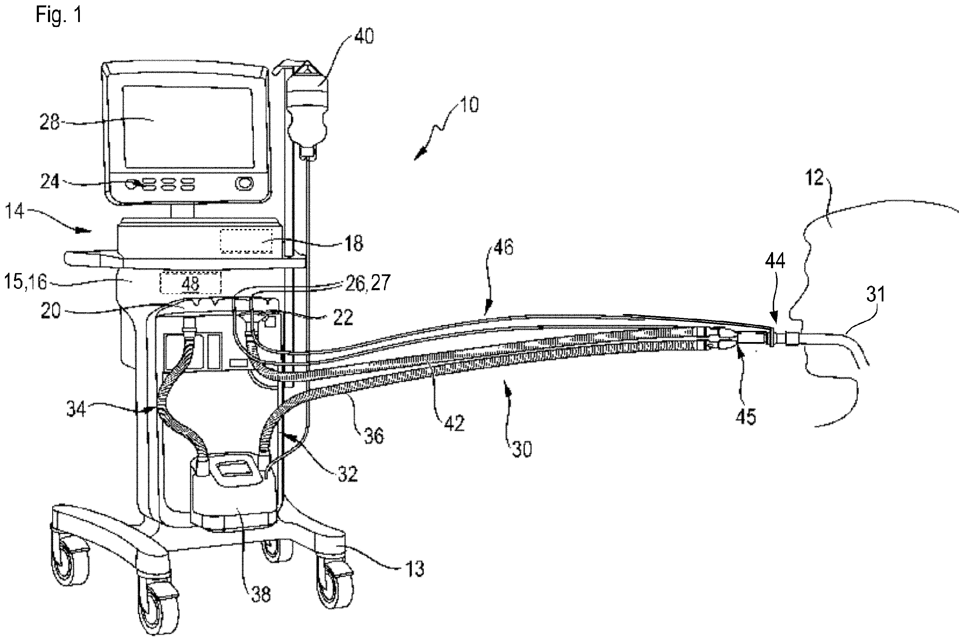

Zur Erläuterung der erfindungsgemäßen Exspirationsventilanordnung, sei zunächst eine die Exspirationsventilanordnung verwendende Beatmungsvorrichtung anhand von Fig. 1 erläutert:
In Figur 1 ist eine Beatmungsvorrichtung allgemein mit 10 bezeichnet. Die Beatmungsvorrichtung 10 dient im dargestellten Beispiel zur künstlichen Beatmung eines humanen Patienten 12. Die Beatmungsvorrichtung 10 kann als mobile Beatmungsvorrichtung 10 auf einem rollbaren Gestell 13 aufgenommen sein.

Die Beatmungsvorrichtung 10 weist ein Gehäuse 14 auf, in dem - von außen wegen des blickdichten Gehäusematerials nicht erkennbar - eine Druckveränderungsanordnung 16 und eine Steuereinrichtung 18 aufgenommen sein können.

Die Druckveränderungsanordnung 16 ist in an sich bekannter Weise aufgebaut und weist eine Atemgas-Liefervorrichtung 15 in Gestalt einer Pumpe, eines Verdichters oder eines Gebläses auf, die jeweils lastveränderlich ansteuerbar sind und daher nicht nur der Einleitung von Beatmungsgas in die Beatmungsvorrichtung, sondern auch der Änderung des Drucks des eingeleiteten Beatmungsgases dienen. Die Atemgas-Liefervorrichtung 15 kann alternativ auch durch einen Druckbehälter gebildet sein, welcher an das Gehäuse 14 der Beatmungsvorrichtung 10 anschließbar ist. Die Druckveränderungsanordnung 16 kann die Atemgas-Liefervorrichtung 15 und gegebenenfalls zusätzlich - oder im Falle eines unter Druck stehenden Gasvorrats als Atemgas-Liefervorrichtung alternativ - ein Reduzierventil und dergleichen aufweisen. Weiter weist die Beatmungsvorrichtung 10 in an sich bekannter Weise eine Inspirationsventilanordnung 20 und eine Exspirationsventilanordnung 22 auf, welche in Figur 1 durch das Gehäuse 14 der Beatmungsvorrichtung 10 verdeckt sind. Die Exspirationsventilanordnung 22 ist eine Exspirationsventilanordnung gemäß der vorliegenden Erfindung.

Die Steuereinrichtung 18 ist üblicherweise als Computer oder Mikroprozessor realisiert. Sie umfasst eine in Figur 1 nicht dargestellte Datenspeichereinrichtung, um für den Betrieb der Beatmungsvorrichtung 10 notwendige Daten speichern und erforderlichenfalls aufrufen zu können. Die Speichereinrichtung kann bei Netzwerkbetrieb auch außerhalb des Gehäuses 14 gelegen und durch eine Datenübertragungsverbindung mit der Steuereinrichtung 18 verbunden sein. Die Datenübertragungsverbindung kann durch eine Kabel- oder eine Funkstrecke gebildet sein. Um jedoch zu verhindern, dass Störungen der Datenübertragungsverbindung sich auf den Betrieb der Beatmungsvorrichtung 10 auswirken können, ist die Speichereinrichtung bevorzugt in die Steuereinrichtung 18 integriert oder wenigstens im selben Gehäuse 14 wie diese aufgenommen.

Zur Eingabe von Daten in die Beatmungsvorrichtung 10 bzw. genauer in die Steuereinrichtung 18 weist die Beatmungsvorrichtung 10 einen Dateneingang 24 auf, welcher in dem in Figur 1 dargestellten Beispiel durch eine Tastatur repräsentiert ist. Die Steuereinrichtung 18 kann alternativ oder zusätzlich zur dargestellten Tastatur Daten über verschiedene Dateneingänge erhalten, etwa über eine Netzwerkleitung, eine Funkstrecke oder über Sensoranschlüsse 26, auf die weiter unten im Einzelnen eingegangen wird.

Zur Ausgabe von Daten an den behandelnden Therapeuten kann die Beatmungsvorrichtung 10 ein Ausgabegerät 28 aufweisen, im dargestellten Beispiel einen Bildschirm.

Zur künstlichen Beatmung ist der Patient 12 mit der Beatmungsvorrichtung 10, genauer mit der Druckveränderungsanordnung 16 im Gehäuse 14, über eine Beatmungsleitungsanordnung 30 verbunden. Der Patient 12 ist hierfür mit einem endotrachealen Rohr 31 intubiert.

Die Beatmungsleitungsanordnung 30, über welche frisches Beatmungsgas von der Beatmungsgasquelle 15 und der Druckveränderungsanordnung 16 in die Lunge des Patienten 12 geleitet werden kann, weist außerhalb des Gehäuses 14 einen Inspirationsschlauch 32 auf. Der Inspirationsschlauch 32 kann unterbrochen sein und einen ersten Inspirationsteilschlauch 34 und einen zweiten Inspirationsteilschlauch 36 aufweisen, zwischen welchen eine Konditionierungseinrichtung 38 zur gezielten Befeuchtung und gegebenenfalls auch Temperierung des dem Patienten 12 zugeführten frischen Beatmungsgases vorgesehen sein kann. Die Konditionierungseinrichtung 38 kann mit einem externen Flüssigkeitsvorrat 40 verbunden sein, über den Wasser zur Befeuchtung oder auch ein Medikament, etwa zur Entzündungshemmung oder zur Erweiterung der Atemwege, dem Beatmungsgas zugeführt werden kann. Bei einem Einsatz der vorliegenden Beatmungsvorrichtung 10 als Anästhesie-Beatmungsvorrichtung können auf diese Weise volatile Anästhetika kontrolliert über die Beatmungsvorrichtung 10 an den Patienten 12 abgegeben werden. Die Konditionierungseinrichtung 38 sorgt dafür, dass das frische Beatmungsgas dem Patienten 12 mit einem vorbestimmten Feuchtegehalt, gegebenenfalls unter Zugabe eines Medikamenten-Aerosols, und mit einer vorbestimmten Temperatur zugeleitet wird.

Die Beatmungsleitungsanordnung 30 weist neben der bereits erwähnten Inspirationsventilanordnung 20 die Exspirationsventilanordnung 22 und weiter einen Exspirationsschlauch 42 auf, über welchen verstoffwechseltes Beatmungsgas aus der Lunge des Patienten 12 in die Atmosphäre abgeblasen wird.

Der Inspirationsschlauch 32 ist mit der Inspirationsventilanordnung 20 gekoppelt, der Exspirationsschlauch 42 ist mit der Exspirationsventilanordnung 22 gekoppelt. Eine etwaige Betätigungssteuerung der Ventilanordnungen 20 und 22 über eine durch die Beatmung erfolgende Selbststeuerung hinaus erfolgt ebenfalls durch die Steuereinrichtung 18.

Während eines Beatmungszyklus ist zunächst für die Dauer der Inspirationsphase die Exspirationsventilanordnung 22 geschlossen und die Inspirationsventilanordnung 20 geöffnet, sodass frisches Beatmungsgas vom Gehäuse 14 zum Patienten 12 nur über den Inspirationsschlauch 32 geleitet werden kann. Eine Strömung des frischen Beatmungsgases wird durch gezielte Druckerhöhung des Beatmungsgases durch die Druckveränderungsanordnung 16 bewirkt. Aufgrund der Druckerhöhung strömt das frische Beatmungsgas in die Lunge des Patienten 12 und expandiert dort den lungennahen Körperbereich, also insbesondere den Brustkorb, gegen die individuelle Elastizität der lungennahen Körperteile. Hierdurch steigt auch der Gasdruck im Inneren der Lunge des Patienten 12 an.

Am Ende der Inspirationsphase wird die Inspirationsventilanordnung 20 geschlossen und die Exspirationsventilanordnung 22 geöffnet. Es beginnt die Exspirationsphase. Aufgrund des bis zum Ende der Inspirationsphase erhöhten Gasdrucks des in der Lunge des Patienten 12 befindlichen Beatmungsgases strömt dieses nach dem Öffnen der Exspirationsventilanordnung 22 durch diese hindurch in die Atmosphäre, wobei sich mit fortschreitender Strömungsdauer der Gasdruck in der Lunge des Patienten 12 verringert. Erreicht der Gasdruck in der Lunge 12 einen an der Beatmungsvorrichtung 10 eingestellten positiven end-exspiratorischen Druck (PEEP), also einen geringfügig höheren Druck als den Atmosphärendruck, wird die Exspirationsphase beendet und es schließt sich ein weiterer Beatmungszyklus an.

Während der Inspirationsphase wird dem Patienten 12 das sogenannte Beatmungs-Tidalvolumen zugeführt, also das Beatmungsgas-Volumen pro Atemhub. Das Beatmungs-Tidalvolumen multipliziert mit der Anzahl an Beatmungszyklen pro Minute, also multipliziert mit der Beatmungsfrequenz, ergibt das Minutenvolumen der vorliegend durchgeführten künstlichen Beatmung.

Bevorzugt ist die Beatmungsvorrichtung 10, insbesondere die Steuereinrichtung 18, dazu ausgebildet, Beatmungs-Betriebsparameter, die den Beatmungsbetrieb der Beatmungsvorrichtung 10 kennzeichnen, während des Beatmungsbetriebs wiederholt zu aktualisieren bzw. zu ermitteln, um sicherzustellen, dass der Beatmungsbetrieb zu jedem Zeitpunkt möglichst optimal auf den jeweils zu beatmenden Patienten 12 abgestimmt ist. Besonders vorteilhaft erfolgt die Bestimmung eines oder mehrerer Beatmungs-Betriebsparameter mit der Beatmungsfrequenz, sodass für jeden Beatmungszyklus aktuelle und damit optimal an den Patienten 12 angepasste Beatmungs-Betriebsparameter bereitgestellt werden können.

Hierzu ist die Beatmungsvorrichtung 10 mit einem oder mehreren Sensoren datenübertragungsmäßig verbunden, welche den Zustand des Patienten oder/und den Betrieb der Beatmungsvorrichtung überwachen.

Einer dieser Sensoren ist ein proximaler Durchflusssensor 44, welcher auf der dem Patienten 12 näher gelegenen Seite eines Y-Verbindungsstücks 45 angeordnet ist und dort die in der Beatmungsleitungsanordnung 30 herrschende Beatmungsgas-Strömung erfasst. Der Durchflusssensor 44 kann mittels einer Sensor-Leitungsanordnung 46 mit den Dateneingängen 26 der Steuereinrichtung 18 gekoppelt sein. Die Sensor-Leitungsanordnung 46 kann, muss jedoch nicht, elektrische Signalübertragungsleitungen umfassen. Sie kann ebenso Schlauchleitungen aufweisen, die den in Strömungsrichtung beiderseits des Durchflusssensors 44 herrschenden Gasdruck an die Dateneingänge 26 übertragen, wo diese von Drucksensoren 27 quantifiziert werden. Der Durchflusssensor 44 ist zwar bevorzugt ein nach dem Differenzdruck-Prinzip arbeitender Durchflusssensor, kann jedoch auch ein nach einem anderen physikalischen Wirkprinzip arbeitender Durchflusssensor sein.

Im Gehäuse 14 ist ein weiterer Durchflusssensor 48 vorgesehen, welcher aufgrund seiner größeren Entfernung vom Patienten 12 - verglichen mit dem proximalen Durchflusssensor 44 - als distaler Durchflusssensor 48 bezeichnet ist.

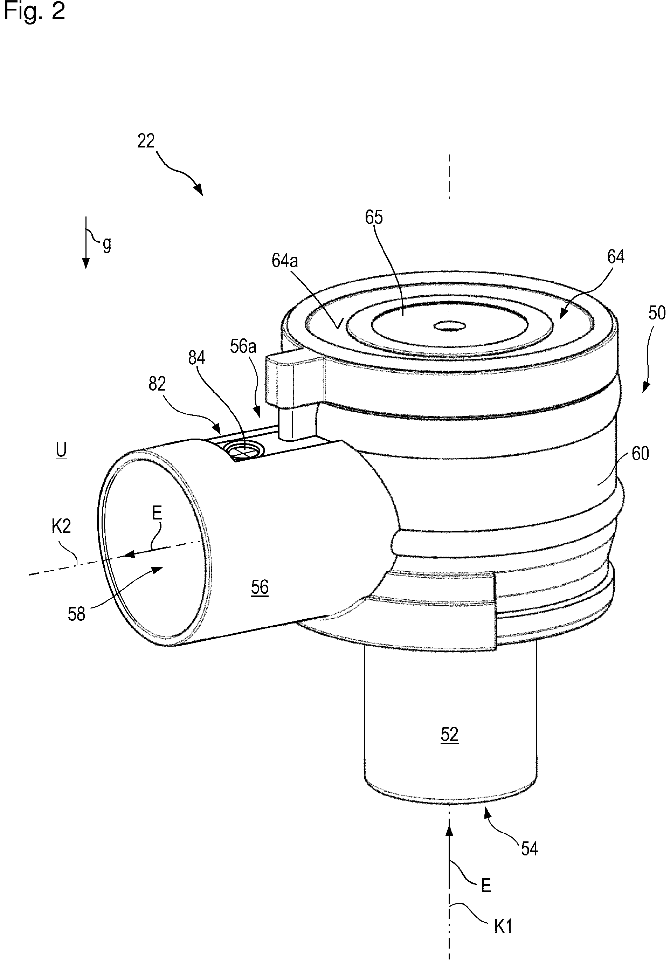

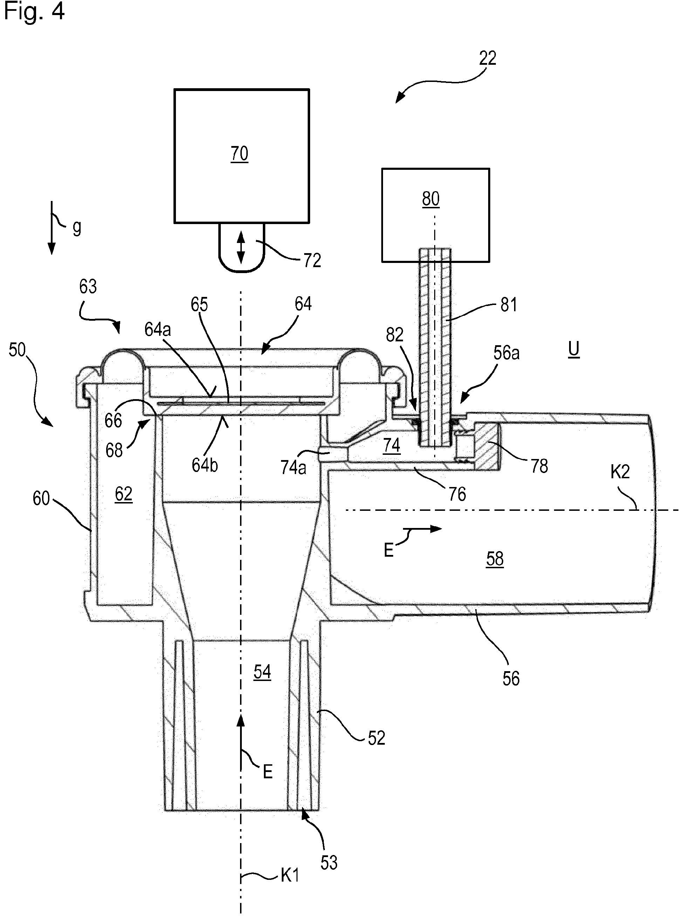

In Figur 2 ist die Exspirationsventilanordnung 22 aus dem Gehäuse 14 der Beatmungsvorrichtung 10 ausgebaut in perspektivischer Darstellung gezeigt. In Figur 4 ist die Exspirationsventilanordnung 22 in einem Längsschnitt grobschematisch dargestellt.

Nachfolgend wird die Exspirationsventilanordnung 22 anhand der Figuren 2 und 4 näher erläutert werden.

Die Exspirationsventilanordnung 22 umfasst ein im Wesentlichen einstückig durch Spritzgießen hergestelltes Kanalbauteil 50 mit einem stromaufwärtigen Exspirationsrohr 52, welches einen stromaufwärtigen Atemgaskanal 54 nach radial außen begrenzt, und mit einem stromabwärtigen Exspirationsrohr 56, welches einen stromabwärtigen Atemgaskanal 58 einfasst. Der stromaufwärtige Atemgaskanal 54 erstreckt sich längs der als geradlinige Kanalachse ausgestalteten ersten Kanalbahn K1, der stromabwärtige Atemgaskanal 58 erstreckt sich längs der ebenfalls als geradlinige Kanalbahn K2 ausgebildeten zweiten Kanalbahn.

Die Exspirationsventilanordnung 22 ist längs der Exspirationsströmungsrichtung E von exspiratorischem Atemgas durchströmbar.

Ein stromabwärtiger Endabschnitt des stromaufwärtigen Exspirationsrohrs 52 ist von einem Ringkammerrohr 60 radial außen umgeben, welches eine Ringkammer 62 nach radial außen begrenzt. Die Ringkammer 62 ist nach radial innen durch das stromaufwärtige Atemgasrohr 52 begrenzt.

Die Exspirationsventilanordnung 22 weist im Bereich des stromabwärtigen Endes des stromaufwärtigen Exspirationsrohrs 52 eine Ventilbaugruppe 63 auf (siehe Figur 4), mit einem Membran-Ventilkörper 64 und weiter mit einem Ventilsitz 66.

Der Membran-Ventilkörper 64 ist an dem dem stromabwärtigen Längsende des stromaufwärtigen Exspirationsrohrs 52 näher gelegenen Längsende des Ringkammerrohrs 60 gehaltert und weist auf seiner dem stromaufwärtigen Exspirationsrohr 52 zugewandten Seite 64b eine Ventilsitzfläche auf, mit welcher der Membran-Ventilkörper 64 auf dem am stromabwärtigen Längsende des stromaufwärtigen Exspirationsrohrs 52 ausgebildeten Ventilsitz 66 aufliegen kann.

In der betriebsbereiten Einbaulage der Exspirationsventilanordnung 22 verläuft die erste Kanalbahn K1 parallel zur Schwerkraftwirkungsrichtung g, sodass der Membran-Ventilkörper 64 durch die Schwerkraft in die in Figur 4 gezeigte Schließstellung hin vorbelastet ist, in welcher der Membran-Ventilkörper 64 auf dem Ventilsitz 66 aufliegt und den stromaufwärtigen Atemgaskanal 54 vom stromabwärtigen Atemgaskanal 58 strömungstechnisch trennt.

Der stromabwärtige Atemgaskanal 58 öffnet in die Außenumgebung U. Sie bildet eine Atemgas-Senke der Beatmungsvorrichtung 10 sowie der Exspirationsventilanordnung 22. Dadurch wird dem exspiratorischen Atemgas vom stromabwärtigen Längsende des stromabwärtigen Exspirationsrohrs 56 her der Umgebungsdruck aufgeprägt.

Dann, wenn -wie es für einen Exspirationsvorgang typisch ist - im stromaufwärtigen Atemgaskanal 54 ein Überdruck bezüglich des Umgebungsdrucks herrscht, wobei der Umgebungsdruck im Übrigen auch auf die vom Ventilsitz 66 abgewandte Seite 64a des Membran-Ventilkörpers 64 wirkt, wird der Membran-Ventilkörper 64 vom Ventilsitz 66 im Wesentlichen längs der ersten Kanalbahn K1 abgehoben, sodass zwischen der dem Ventilsitz 66 zugwandten Seite 64b des Membran-Ventilkörpers 64 und dem Ventilsitz 66 ein Ringspalt 68 entsteht, dessen Spalthöhe in Figur 4 noch 0 beträgt.

Bei geöffneter Ventilbaugruppe 63 strömt daher exspiratorisches Atemgas getrieben durch den relativen Überdruck im stromaufwärtigen Atemgaskanal 54 durch den Ventilspalt 68 in die Ringkammer 62 und von dort in den stromabwärtigen Atemgaskanal 58 und schließlich in die Außenumgebung U.

Zur besseren Orientierung sei angemerkt, dass das stromaufwärtige Exspirationsrohr 52 an seinem dem Ventilsitz 66 entgegengesetzten stromaufwärtigen Längsendbereich eine Verbindungsformation 53 aufweist, mit welcher das stromaufwärtige Exspirationsrohr 52 und damit die Exspirationsventilanordnung 22 mit dem Exspirationsschlauch 42 aus Figur 1 verbindbar ist.

Der im stromaufwärtigen Atemgaskanal 54 herrschende relative Überdruck bezüglich des Gasdrucks im stromabwärtigen Atemgaskanal 58 nimmt mit zunehmender Dauer des Exspirationsvorgangs ab. Dabei ist es wichtig, einen positiven end-exspiratorischen Druck (PEEP) aufrechtzuerhalten, um der Patientenlunge bis zum Ende des Exspirationsvorgangs einen positiven Gegendruck entgegenzuhalten und so einem Kollabieren der künstlich beatmeten Patientenlunge vorzubeugen.

Die Einstellung des PEEP erfolgt durch Einstellung der Spalthöhe des Ventilspalts 68. Hierzu umfasst die Exspirationsventilanordnung 22 einen Aktuator 70 (nur in Figur 4 dargestellt), etwa einen elektromagnetischen Aktuator 70, aus welchem ein Stößel 72 durch die Steuereinrichtung 18 der Beatmungsvorrichtung 10 ausfahrbar und wieder einziehbar ist.

Der Stößel 72 ist mit seinem vom übrigen Aktuator 70 fern liegenden und bei einer Ausfahrbewegung vorauseilenden Längsende mit einer starren Platte 65 im Membran-Ventilkörper 64 in Anlageeingriff bringbar, sodass der Membran-Ventilkörper 64 durch den Aktuator 70 definiert zum Ventilsitz 66 hin bewegbar ist. Ein Abheben des Membran-Ventilkörpers 64 vom Ventilsitz 66 ist dagegen durch den Stößel 72 bzw. durch den Aktuator 70 nicht vorgesehen, da mit dem Anlageeingriff zwischen Stößel 72 und starrer Platte 65 nur Druckkräfte, jedoch keine Zugkräfte übertragen werden können.

Für eine genaue Einstellung des positiv end-exspiratorischen Drucks durch den Aktuators 70 ist die Kenntnis des im stromaufwärtigen Atemgaskanal 54 möglichst nahe beim Ventilsitz 66 herrschenden Atemgasdrucks hilfreich.

Hierzu weist die erfindungsgemäße Exspirationsventilanordnung 22 innerhalb des stromabwärtigen Exspirationsrohrs 56 eine Bypass-Kammer 74 auf (siehe Figur 4). Diese Bypass-Kammer 74 kommuniziert strömungstechnisch an ihrem Längsende 74a mit dem stromaufwärtigen Atemgaskanal 54. Dadurch herrscht auch in der Bypass-Kammer 74 der gleiche Gasdruck wie am stromabwärtigen Ende des stromaufwärtigen Atemgaskanals 54.

Eine Trennwand 76, welche die Bypass-Kammer vom stromabwärtigen Atemgaskanal 58 trennt, ist vorzugsweise einstückig mit den Exspirationsrohren 52 und 56 sowie mit dem Ringkammerrohr 60 ausgebildet. Dadurch wird eine kompakte Anordnung erhalten, von welcher sich keine Bauteile oder Bauteilabschnitte lösen können.

Wegen der einstückigen Ausbildung der Trennwand 76 mit dem Kanalbauteil 50 muss eine Öffnung der Bypass-Kammer 74, welche bei der spritzgusstechnischen Herstellung durch einen Schieber belegt ist, an der fertigen Exspirationsventilanordnung 22 durch einen Stopfen 78 verschlossen sein. Der Stopfen 78 kann reibschlüssig oder durch Verklebung oder durch Verschweißung mit dem übrigen Kanalbauteil 50 verbunden sein.

Ein im Einbauzustand der Exspirationsventilanordnung 22 vorzugsweise entgegen der Schwerkraftwirkungsrichtung g weisender Sensor-Ankopplungswandabschnitt 56a des am Ort der Ausbildung des Sensor-Ankopplungswandabschnitts 56a sowohl den stromabwärtigen Atemgaskanal 58 als auch die Bypass-Kammer 74 einfassenden stromabwärtigen Exspirationsrohrs 56 ist zur messtechnischen Ankopplung eines Gasdrucksensors 80 (siehe Figur 4) ausgebildet.

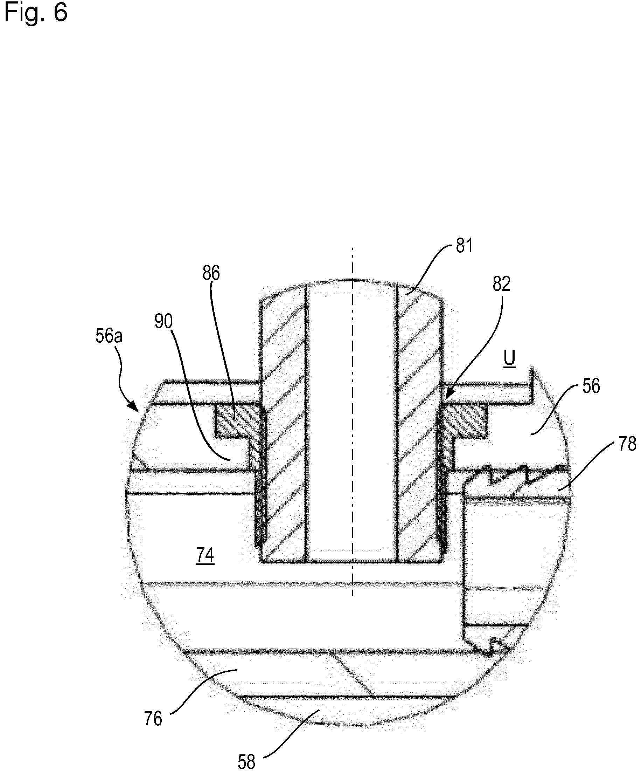

In den Figuren 4 und 6 ist der Gasdrucksensor 80 messtechnisch an die Bypass-Kammer und damit an den stromaufwärtigen Atemgaskanal 54 angekoppelt dargestellt, in den Figuren 2, 3 und 5 ist die Exspirationsventilanordnung 22 lediglich zur Ankopplung eines Gasdrucksensors ausgebildet, jedoch ohne Realisierung dieser Ankopplung dargestellt.

Daraus ergibt sich, dass die Exspirationsventilanordnung 22 sowohl mit messtechnisch über die Bypass-Kammer 74 an den stromaufwärtigen Atemgaskanal 54 angekoppeltem Gasdrucksensor 80 als auch ohne derartige Ankopplung betrieben werden kann.

Im Sensor-Ankopplungswandabschnitt 56a, welcher zur erleichterten Bearbeitung auf seiner radial vom stromabwärtigen Atemgaskanal 58 sowie von der Bypass-Kammer 74 weg weisenden Seite bevorzugt mit ebener Außenfläche ausgebildet ist, ist eine Sensor-Ankopplungsöffnung 82 ausgebildet, welche den Sensor-Ankopplungswandabschnitt 56a in Dickenrichtung des stromabwärtigen Exspirationsrohrs 56 vollständig durchsetzt. Diese Sensor-Ankopplungsöffnung 82 ist zunächst durch eine Verschluss-Membran 84 verschlossen (siehe Figuren 2, 3 und 5). Die Verschluss-Membran 84 kann jedoch in einfacher Weise durchstochen und somit die Bypass-Kammer 74 von der Außenumgebung U her zugänglich gemacht werden. Beispielsweise ist in den Figuren 4 und 6 dargestellt, wie die ursprünglich die Sensor-Ankopplungsöffnung 82 überspannende Verschluss-Membran 84 durch ein Ankopplungsrohr 81 durchstoßen wurde, welches den Innenbereich der Bypass-Kammer 74 messtechnisch mit dem Gasdrucksensor 80 verbindet.

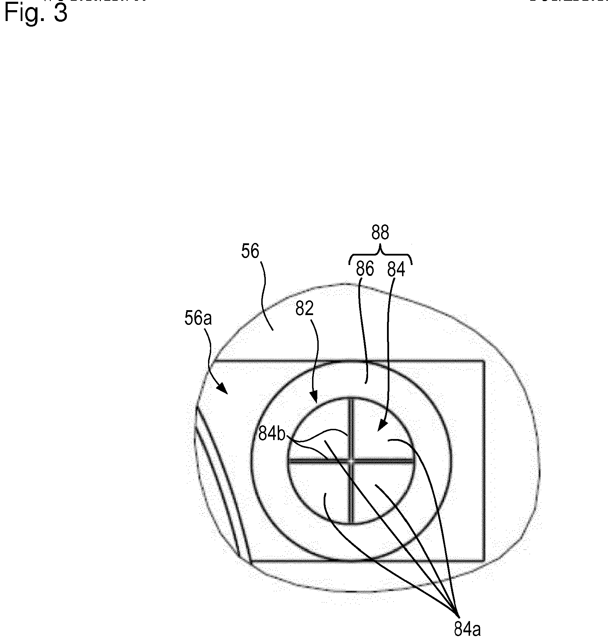

Wie in Figur 3 zu erkennen ist, kann die Verschluss-Membran 84 von einem einstückig mit der Verschluss-Membran 84 zusammenhängenden Randbereich 86 eingefasst sein, welcher mit dem Sensor-Ankopplungswandabschnitt 56a beispielsweise durch Verklebung unlösbar verbunden ist. Die Verschluss-Membran 84 und der Randabschnitt 86 bilden dann einen die Sensor-Ankopplungsöffnung 82 verschließenden Verschluss 88.

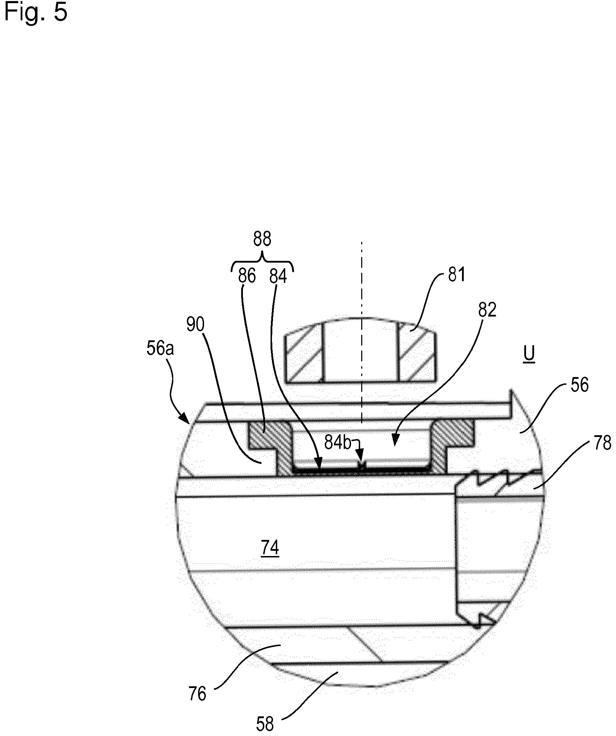

Wie insbesondere in Figur 5 zu erkennen ist, ist der Randabschnitt 86 des Verschlusses 88 mit einem die Sensor-Ankopplungsöffnung 82 umgebenden Grenzbereich 90 des Sensor-Ankopplungswandabschnitts 56a verklebt.

Im ungeöffneten Zustand weist die Membran 84 einen Normalbereich 84a sowie einen Materialschwächungsbereich 84b auf, welcher eine geringere Membranwandstärke aufweist als der Normalbereich 84a.

Der Materialschwächungsbereich 84b umfasst, wie in Figur 3 beispielhaft dargestellt ist, zwei sich unter einem rechten Winkel schneidende Durchmesserlinien, längs welchen die Membran 84 eine geringere Wandstärke aufweist als in den Bereichen beiderseits dieser Linien. Somit ist der Normalbereich 84a in vier gleich große Teilbereiche unterteilt, von welchen jeder jeweils einen Quadranten der im Beispiel kreisförmigen Membran 84 belegt. Jede Durchmesserlinie des Materialschwächungsbereichs 84b unterteilt dabei die Membran 84 in etwa zwei gleich große Flächenbereiche.

Durch die Ausbildung des Materialschwächungsbereichs 84b reißt die Membran 84 beim Durchstoßen durch das Ankopplungsrohr 81, wie es in den Figuren 4 bis 6 gezeigt ist, entlang der Soll-Reißstellen bildenden Linien des Materialschwächungsbereichs 84b, sodass die Membran 84 beim Durchstoßen mit einem Ankopplungsrohr 81 in vorbestimmter Weise öffnet.

Wie in Figur 6 gezeigt ist, kann sowohl der Randbereich 86 des Verschlusses 88 als auch die Membranreste eine das Ankopplungsrohr 81 an der Stelle der durchsetzten Sensor-Ankopplungsöffnung 82 nach radial außen abdichten und somit Gas-Fehlströmungen zwischen dem Inneren der Bypass-Kammer 74 und der Außenumgebung U und damit eine Verfälschung des vom Gasdrucksensor 80 ermittelten Gasdrucks im stromaufwärtigen Atemgaskanal 54 verhindern.

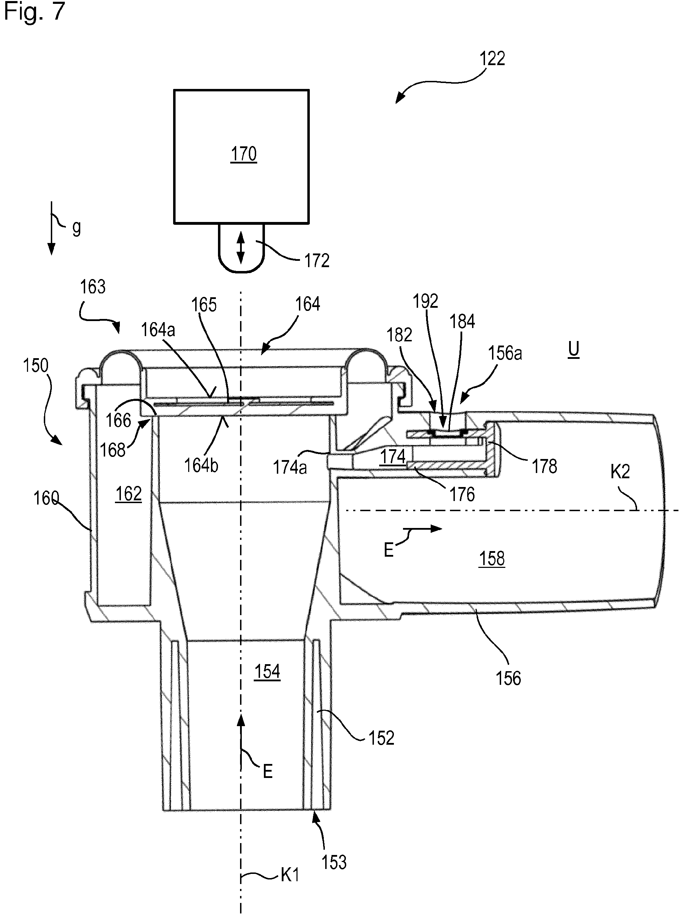

Figur 7 zeigt in gleicher Perspektive und Lage der Schnittebene wie in Figur 2 eine zweite Ausführungsform einer erfindungsgemäßen Exspirationsventilanordnung. Gleiche und funktionsgleiche Bauteile und Bauteilabschnitte wie in der ersten Ausführungsform sind in der zweiten Ausführungsform mit gleichen Bezugszeichen versehen wie in der ersten Ausführungsform der Figuren 2 bis 6, jedoch erhöht um die Zahl 100.

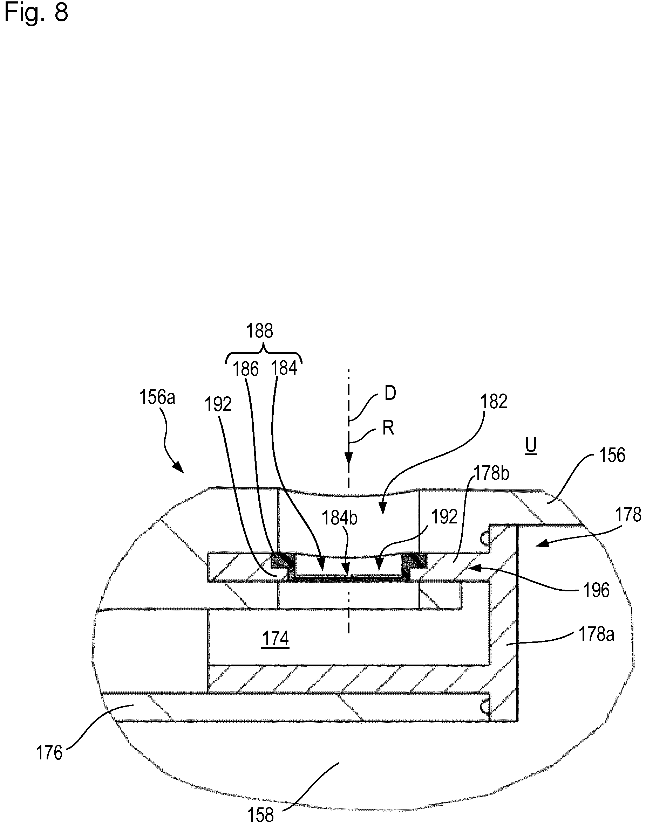

Die zweite Ausführungsform wird nachfolgend nur insoweit beschrieben, als sie sich von der ersten Ausführungsform unterscheidet, auf deren Beschreibung ansonsten auch zur Erläuterung der zweiten Ausführungsform der Figuren 7 und 8 verwiesen wird.

Bei der in den Figuren 7 und 8 dargestellten zweiten Ausführungsform ist der Verschluss 188 (s. Figur 8) am Stopfen 178 als einem mit dem Sensor-Ankopplungswandabschnitt 156a zusammenwirkenden Zusatzbauteil vorgesehen. Der Stopfen 178 weist dabei an einem von einem Stopfenkopf 178a abstehenden Befestigungsschenkel, etwa einer geschlossen umlaufenden Befestigungshülse 178b eine Ergänzungsöffnung 192 auf, welche mit der Sensor-Ankopplungsöffnung 182, bevorzugt mit koaxialen die jeweiligen Öffnungen 182 und 192 zentral durchsetzenden Öffnungsachsen, überlappt. Der die Verschluss-Membran 184 einfassende Randbereich 186 ist im vorliegenden zweiten Ausführungsbeispiel mit einem die Ergänzungsöffnung 192 einfassenden Einfassungsbereich 194 verbunden, beispielsweise durch 2-Komponenten-Spritzguss.

Im betriebsbereiten Zustand der Exspirationsventilanordnung 122 fasst der Befestigungsschenkel, insbesondere in Gestalt der geschlossen umlaufenden Befestigungshülse 178b, einen Teil des Innenvolumens der Bypass-Kammer 174 ein.

Der die Ergänzungsöffnung 192 aufweisende Abschnitt des Stopfens 178 kann in einer diesen Stopfenabschnitt längs einer Durchdringungsachse D der Ergänzungsöffnung 192 beiderseits begrenzenden Aufnahmeformation 196 im Sensor-Ankopplungsabschnitt 156a aufgenommen sein. Der die Ergänzungsöffnung 192 aufweisende Stopfenabschnitt kann jedoch, wie im ersten Ausführungsbeispiel, auch nur radial außen von Material des stromabwärtigen Exspirationsrohrs 156 bzw. zwischen Trennwand 176 und Sensor-Ankopplungsabschnitt 156a aufgenommen sein.

Die Sensor-Ankopplungsöffnung 182 ist vorzugsweise nicht kleiner als die Ergänzungsöffnung 192 und weist ebenso bevorzugt die gleiche Gestalt auf. Die Sensor-Ankopplungsöffnung 182 kann geringfügig größer als oder gleich groß wie die Ergänzungsöffnung 192 sein, insbesondere, wenn in Durchdringungsrichtung R längs der Durchdringungsachse D hinter dem die Ergänzungsöffnung 192 aufweisenden Stopfenabschnitt noch Material des Sensor-Ankopplungswandabschnitts 156a gelegen ist. Wenigstens der in Durchdringungsrichtung R hinter der Ergänzungsöffnung 192 gelegene Teil der Sensor-Ankopplungsöffnung 182 kann größer sein als die Ergänzungsöffnung 192, um den nach dem Durchstechen der Verschluss-Membran 184 in Durchdringungsrichtung R vom Randbereich 186 abstehenden Membranresten einen Aufnahmeraum zwischen dem Sensor-Ankopplungswandabschnitt 156a und einem die Sensor-Ankopplungsöffnung 182 und die Ergänzungsöffnung 192 durchsetzenden Ankopplungsrohr bereitzustellen.

## Patentansprüche

1. Exspirationsventilanordnung (22; 122) für eine Exspirationsleitung einer Beatmungsvorrichtung (10) zur künstlichen Beatmung von Patienten, wobei die Exspirationsventilanordnung (22; 122) in einer Exspirationsströmungsrichtung (E) durchströmbar ist und umfasst:
- einen stromaufwärtigen Atemgaskanal (54; 154), welcher sich längs einer ersten Kanalbahn (K1) erstreckt und welcher mit einem vom Patienten kommenden Abschnitt (42) der Exspirationsleitung verbunden oder verbindbar ist,
- einen stromabwärtigen Atemgaskanal (58; 158), welcher sich längs einer zweiten Kanalbahn (K2) erstreckt und welcher mit einer Atemgas-Senke (U), wie etwa der Außenumgebung (U), verbunden oder verbindbar ist,
- eine einen Ventilkörper (64; 164) und einen Ventilsitz (66; 166) aufweisende Ventilbaugruppe (63; 163), welche zwischen dem stromaufwärtigen und dem stromabwärtigen Atemgaskanal (54, 58; 154, 158) derart vorgesehen ist, dass sie bei einem vorbestimmten ersten Atemgasüberdruck im stromaufwärtigen Atemgaskanal (54; 154) relativ zum stromabwärtigen Atemgaskanal (58; 158) eine exspiratorische Atemgasströmung vom stromaufwärtigen Atemgaskanal (54; 154) in den stromabwärtigen Atemgaskanal (58; 158) zulässt und dass sie bei einem vorbestimmten zweiten Atemgasüberdruck im stromabwärtigen Atemgaskanal (58; 158) relativ zum stromaufwärtigen Atemgaskanal (54; 154) eine Gasströmung vom stromabwärtigen Atemgaskanal (58; 158) in den stromaufwärtigen Atemgaskanal (54; 154) sperrt,
wobei die Exspirationsventilanordnung (22; 122) eine Bypass-Kammer (74; 174) aufweist, welche strömungsmechanisch mit dem stromaufwärtigen Atemgaskanal (54; 154) kommuniziert und zur Ankopplung eines Gasdrucksensors (80) ausgebildet
ist,
**dadurch gekennzeichnet, dass**
die Bypass-Kammer (74; 174) sich ausgehend vom stromaufwärtigen Atemgaskanal (54; 154) in den Bereich des stromabwärtigen Atemgaskanals (58; 185) erstreckt und dort zur Ankopplung des Gasdrucksensors (80) ausgebildet ist.

2. Exspirationsventilanordnung (22; 122) nach Anspruch 1,
**dadurch gekennzeichnet, dass** ein Sensor-Ankopplungswandabschnitt (56a; 156a) einer die Bypass-Kammer (74; 174) begrenzenden Wandung eine Sensor-Ankopplungsöffnung (82; 182) aufweist, welche den Sensor-Ankopplungswandabschnitt (56a; 156a) der Bypass-Kammer (74; 174) durchsetzt, wobei die Sensor-Ankopplungsöffnung (82; 182) durch einen offenbaren Verschluss (88; 188) verschlossen ist.

3. Exspirationsventilanordnung (22; 122) nach Anspruch 2,
**dadurch gekennzeichnet, dass** der Verschluss (88; 188) eine durchstechbare Verschluss-Membran (84; 184) umfasst, welche aus einem im Vergleich zum Material des umgebenden Sensor-Ankopplungswandabschnitts (56a; 156a) weicheren Material gebildet ist.

4. Exspirationsventilanordnung (22; 122) nach Anspruch 3,
**dadurch gekennzeichnet, dass** die Verschluss-Membran (84; 184) aus einem Elastomer, insbesondere aus Natur- oder Silikonkautschuk, gebildet ist.

5. Exspirationsventilanordnung (22; 122) nach Anspruch 3 oder 4,
**dadurch gekennzeichnet, dass** die Verschluss-Membran (84; 184) einen Normalbereich (84a) und einen vom Normalbereich (84a) verschiedenen Materialschwächungsbereich (84b; 184b) aufweist, in welchem die Reißfestigkeit der Verschluss-Membran (84; 184), verglichen mit dem Normalbereich (84a), vermindert ist.

6. Exspirationsventilanordnung (22; 122) nach Anspruch 5,
**dadurch gekennzeichnet, dass** die Verschluss-Membran (84; 184) im Materialschwächungsbereich (84b; 184b) eine geringere Materialdicke aufweist als im Normalbereich (84a).

7. Exspirationsventilanordnung (22; 122) nach Anspruch 5 oder 6,
**dadurch gekennzeichnet, dass** der Materialschwächungsbereich (84b; 184b) musterartig in der Verschluss-Membran (84; 184) ausgebildet ist.

8. Exspirationsventilanordnung (22; 122) nach einem der vorhergehenden Ansprüche,
**dadurch gekennzeichnet, dass** ein Abschnitt einer den stromabwärtigen Atemgaskanal einfassenden Wandung (56; 156) den Sensor-Ankopplungswandabschnitt (56a; 156a) der Bypass-Kammer (74; 174) bildet.

9. Exspirationsventilanordnung (22; 122) nach einem der vorhergehenden Ansprüche,
**dadurch gekennzeichnet, dass** die Bypass-Kammer (74; 174) im stromabwärtigen Atemgaskanal (58; 158) verläuft.

10. Exspirationsventilanordnung (22; 122) nach Anspruch 9,
**dadurch gekennzeichnet, dass** eine die Bypass-Kammer (74; 174) vom stromabwärtigen Atemgaskanal (58; 158) trennende Trennwand (76; 176) einstückig mit der den stromabwärtigen Atemgaskanal (58; 158) einfassenden Wandung (56; 156) ausgebildet ist.

11. Exspirationsventilanordnung (22; 122) nach Anspruch 10,
**dadurch gekennzeichnet, dass** ein den stromaufwärtigen Atemgaskanal (54; 154) einfassendes erstes Exspirationsrohr (52; 152), ein den stromabwärtigen Atemgaskanal (58; 158) einfassendes zweites Exspirationsrohr (56; 156) und die die Bypass-Kammer (74; 174) vom stromabwärtigen Atemgaskanal (58; 158) trennende Trennwand (76; 176) einstückig als Kanalbauteil (50; 150) ausgebildet sind.

12. Exspirationsventilanordnung (22; 122) nach einem der vorhergehenden Ansprüche,
**dadurch gekennzeichnet, dass** der Ventilkörper (64; 164) ein ein Längsende des stromaufwärtigen Atemgaskanals (54; 154) überspannender Membran-Ventilkörper (64; 164) ist und dass der Ventilsitz (66; 166) an dem Längsende des stromaufwärtigen Atemgaskanals (54; 154) ausgebildet ist.

13. Exspirationsventilanordnung (22; 122) nach Anspruch 12, unter Einbeziehung des Anspruchs 3,
**dadurch gekennzeichnet, dass** der Membran-Ventilkörper (64; 164) und die Verschluss-Membran (84; 184) mit zueinander parallelen Membranflächen an der Exspirationsventilanordnung (22; 122) vorgesehen sind.

14. Exspirationsventilanordnung (22; 122) nach Anspruch 13,
**dadurch gekennzeichnet, dass** die Verschluss-Membran (84; 184) bei betriebsbereit angeordneter Exspirationsventilanordnung (22; 122) orthogonal zur Schwerkraftwirkungsrichtung (g) angeordnet ist, wobei ihre von der Bypass-Kammer (74; 174) wegweisende Außenseite entgegen der Schwerkraftwirkungsrichtung (g) weist.

15. Beatmungsvorrichtung (10) zur künstlichen Beatmung von Patienten mit einer Atemgas-Liefervorrichtung (15), von welcher eine Inspirationsleitung zu einer Patienten-Beatmungsschnittstelle (31) führt, von welcher wiederum eine Exspirationsleitung zu einer Atemgas-Senke (U), wie etwa der Umgebungsatmosphäre (U), führt,
**dadurch gekennzeichnet, dass** in der Exspirationsleitung eine Exspirationsventilanordnung (22; 122) nach einem der vorhergehenden Ansprüche vorgesehen ist, wobei der stromaufwärtige Atemgaskanal (54; 154) durch einen Abschnitt (42) der Exspirationsleitung mit der Patienten-Beatmungsschnittstelle (31) zur Übertragung von exspiratorischem Atemgas von der Patienten-Beatmungsschnittstelle (31) verbunden ist.

## Claims

1. An exhalation valve arrangement (22; 122) for an exhalation line of a ventilation apparatus (10) for artificial ventilation of patients, the exhalation valve arrangement (22; 122) being flow-capable in an exhalation flow direction (E) and encompassing:
- an upstream respiratory gas conduit (54; 154) that extends along a first conduit path (K1) and is connected or connectable to a portion (42), coming from the patient, of the exhalation line;
- a downstream respiratory gas conduit (58; 158) that extends along a second conduit path (K2) and is connected or connectable to a respiratory gas sink (U), for example the external environment (U);
- a valve subassembly (63; 163) which comprises a valve body (64; 164) and a valve seat (66; 166) and which is provided between the upstream and the downstream respiratory gas conduit (54, 58; 154, 158) in such a way that, in the context of a predetermined first respiratory gas overpressure in the upstream respiratory gas conduit (54; 154) relative to the downstream respiratory gas conduit (58; 158), it permits an expiratory respiratory gas flow from the upstream respiratory gas conduit (54; 154) into the downstream respiratory gas conduit (58; 158); and in the context of a predetermined second respiratory gas overpressure in the downstream respiratory gas conduit (58; 158) relative to the upstream respiratory gas conduit (54; 154), it blocks a gas flow from the downstream respiratory gas conduit (58; 158) into the upstream respiratory gas conduit (54; 154),
wherein the exhalation valve arrangement (22; 122) comprises a bypass chamber (74; 174) which communicates in terms of flow mechanics with the upstream respiratory gas conduit (54; 154) and is embodied for attachment of a gas pressure sensor (80),
**characterized in that** the bypass chamber (74; 174) extends, proceeding from the upstream respiratory gas conduit (54; 154), into the region of the downstream respiratory gas conduit (58; 158) is embodied there for attachment of the gas pressure sensor (80).

2. The exhalation valve arrangement (22; 122) according to Claim 1,
**characterized in that** a sensor attachment wall portion (56a; 156a) of a wall that delimits the bypass chamber (74; 174) comprises a sensor attachment opening (82; 182) that passes through the sensor attachment wall portion (56a; 156a) of the bypass chamber (74; 174), the sensor attachment opening (82; 182) being closed off by an openable closure (88; 188).

3. The exhalation valve arrangement (22; 122) according to Claim 2,
**characterized in that** the closure (88; 188) encompasses a puncturable closure membrane (84; 184) that is constituted from a material that is softer compared with the material of the encircling sensor attachment wall portion (56a; 156a).

4. The exhalation valve arrangement (22; 122) according to Claim 3,
**characterized in that** the closure membrane (84; 184) is constituted from an elastomer, in particular from natural or silicone rubber.

5. The exhalation valve arrangement (22; 122) according to Claim 3 or 4,
**characterized in that** herein the closure membrane (84; 184) comprises a normal region (84a) and a material weakening region (84b, 184b), different from the normal region (84a), in which the tearing strength of the closure membrane (84; 184) is decreased compared with the normal region (84a).

6. The exhalation valve arrangement (22; 122) according to Claim 5,
**characterized in that** the closure membrane (84; 184) has a thinner material thickness in the material weakening region (84b; 184b) than in the normal region (84a).

7. The exhalation valve arrangement (22; 122) according to Claim 5 or 6,
**characterized in that** the material weakening region (84b; 184b) is embodied in patterned fashion in the closure membrane (84; 184).

8. The exhalation valve arrangement (22; 122) according to one of the preceding claims,
**characterized in that** a portion of a wall (56; 156) that surrounds the downstream respiratory gas conduit forms the sensor attachment wall portion (56a; 156a) of the bypass chamber (74; 174).

9. The exhalation valve arrangement (22; 122) according to one of the preceding claims,
**characterized in that** the bypass chamber (74; 174) extends in the downstream respiratory gas conduit (58; 158).

10. The exhalation valve arrangement (22; 122) according to Claim 9,
**characterized in that** a partition (76; 176) that separates the bypass chamber (74; 174) from the downstream respiratory gas conduit (58; 158) is embodied in one piece with the wall (56; 156) that surrounds the downstream respiratory gas conduit (58; 158).

11. The exhalation valve arrangement (22; 122) according to Claim 10,
**characterized in that** a first exhalation tube (52; 152) that surrounds the upstream respiratory gas conduit (54; 154), a second exhalation tube (56; 156) that surrounds the downstream respiratory gas conduit (58; 158), and the partition (76; 176) that separates the bypass chamber (74; 174) from the downstream respiratory gas conduit (58; 158) are embodied in one piece as a conduit component (50; 150).

12. The exhalation valve arrangement (22; 122) according to one of the preceding claims,
**characterized in that** the valve body (64; 164) is a membrane valve body (64; 164) that spans a longitudinal end of the upstream respiratory gas conduit (54; 154); and the valve seat (66; 166) is embodied at the longitudinal end of the upstream respiratory gas conduit (54; 154).

13. The exhalation valve arrangement (22; 122) according to Claim 12, including Claim 3,
**characterized in that** the membrane valve body (64; 164) and the closure membrane (84; 184) are provided with mutually parallel membrane surfaces on the exhalation valve arrangement (22; 122).

14. The exhalation valve arrangement (22; 122) according to Claim 13,
**characterized in that** when the exhalation valve arrangement (22; 122) is arranged in operationally ready fashion, the closure membrane (84; 184) is arranged orthogonally to the direction of gravity (g), such that its outer side, facing away from the bypass chamber (74; 174), faces oppositely to the direction of gravity (g).

15. A ventilation apparatus (10) for artificial ventilation of patients, having a respiratory gas supply apparatus (15) from which an inhalation line leads to a patient ventilation interface (31), from which an exhalation line leads in turn to a respiratory gas sink (U), for example the ambient atmosphere (U),
**characterized in that** an exhalation valve arrangement (22; 122) according to one of the preceding claims is provided in the exhalation line, the upstream respiratory gas conduit (54; 154) being connected by a portion (42) of the exhalation line to the patient ventilation interface (31) in order to transfer expiratory respiratory gas from the patient ventilation interface (31).

## Revendications

1. Agencement de soupape d'expiration (22; 122) pour un conduit d'expiration d'un appareil respiratoire (10) pour la respiration artificielle de patients, l'agencement de soupape d'expiration (22; 122) pouvant être traversé dans un sens d'écoulement d'expiration (E) et comprenant :
- un canal de gaz respiratoire amont (54; 154) qui s'étend le long d'un premier trajet de canal (K1) et qui est relié ou peut être relié à une partie (42) du conduit expiratoire en provenance du patient,
- un canal de gaz respiratoire aval (58; 158) qui s'étend le long d'un deuxième trajet de canal (K2) et qui est relié ou peut être relié à un puits de gaz respiratoire (U), tel que l'environnement extérieur (U),
- un agencement de soupape (63; 163) comprenant un corps de soupape (64; 164) et un siège de soupape (66; 166), ledit agencement de soupape étant prévu entre lesdits canaux de gaz respiratoire amont et aval (54, 58; 154, 158) de telle sorte qu'à une première surpression de gaz respiratoire prédéterminée dans ledit canal de gaz respiratoire amont (54; 154) par rapport au canal de gaz respiratoire aval (58; 158), il permet un écoulement de gaz respiratoire expiratoire du canal de gaz respiratoire amont (54; 154) dans le canal de gaz respiratoire aval (58; 158) et de telle sorte qu'à une seconde surpression de gaz respiratoire prédéterminée dans le canal de gaz respiratoire aval (58; 158) par rapport au canal de gaz respiratoire amont (54; 154) il bloque un écoulement de gaz du canal de gaz respiratoire aval (58; 158) dans le canal de gaz respiratoire amont (54; 154), dans lequel l'agencement de soupape d'expiration (22; 122) comprend une chambre de dérivation (74; 174) qui est en communication fluidique avec le passage de gaz respiratoire amont (54; 154) et qui est adaptée pour un accouplement d'un capteur de pression de gaz (80),
**caractérisé en ce que** la chambre de dérivation (74; 174) s'étend en partant du canal de gaz respiratoire amont (54; 154) dans la zone du canal de gaz respiratoire aval (58; 185) et y est adapté pour l'accouplement du capteur de pression de gaz (80).

2. Agencement de soupape expiratoire (22; 122) selon la revendication 1,
**caractérisé en ce qu'**une partie de paroi d'accouplement de capteur (56a, 156a) d'une paroi définissant la chambre de dérivation (74; 174) comprend une ouverture d'accouplement de capteur (82; 182) s'étendant à travers la partie de paroi d'accouplement de capteur (56a; 156a) de la chambre de dérivation (74; 174), dans lequel l'ouverture d'accouplement de capteur (82; 182) est fermée par une fermeture ouvrable (88; 188).

3. Agencement de soupape expiratoire (22; 122) selon la revendication 2,
**caractérisé en ce que** ladite fermeture (88; 188) comprend un diaphragme de fermeture perçable (84; 184) formé d'un matériau plus souple par rapport au matériau de la partie de paroi d'accouplement de capteur (56a;156a) environnante.

4. Agencement de soupape d'expiration (22; 122) selon la revendication 3,
**caractérisé en ce que** le diaphragme de fermeture (84; 184) est formé d'un élastomère, en particulier de caoutchouc naturel ou de silicone.

5. Agencement de soupape d'expiration (22; 122) selon la revendication 3 ou 4,
**caractérisé en ce que** le diaphragme de fermeture (84; 184) présente une zone normale (84a) et une zone d'affaiblissement du matériau (84b; 184b) différente de la zone normale (84a), dans laquelle la résistance à la déchirure du diaphragme de fermeture (84; 184) est réduite par rapport à la zone normale (84a).

6. Agencement de soupape d'expiration (22; 122) selon la revendication 5,
**caractérisé en ce que** le diaphragme de fermeture (84; 184) présente une épaisseur de matériau plus faible dans la zone d'affaiblissement du matériau (84b; 184b) que dans la zone normale (84a).

7. Agencement de soupape d'expiration (22; 122) selon la revendication 5 ou 6,
**caractérisé en ce que** ladite région d'affaiblissement du matériau (84b; 184b) est modelée dans ledit diaphragme de fermeture (84; 184).

8. Agencement de soupape expiratoire (22; 122) selon l'une des revendications précédentes,
**caractérisé en ce qu'**une partie d'une paroi (56; 156) enfermant le canal de gaz respiratoire aval forme la partie de paroi d'accouplement de capteur (56a; 156a) de la chambre de dérivation (74; 174).

9. Agencement de soupape d'expiration (22; 122) selon l'une des revendications précédentes,
**caractérisé en ce que** ladite chambre de dérivation (74; 174) s'étend dans le canal de gaz respiratoire aval (58; 158).

10. Agencement de soupape d'expiration (22; 122) selon la revendication 9,
**caractérisé en ce qu'**une cloison (76; 176) séparant la chambre de dérivation (74; 174) du canal de gaz respiratoire aval (58; 158) est formée d'une seule pièce avec la paroi (56; 156) enfermant le canal de gaz respiratoire aval (58; 158).

11. Agencement de soupape d'expiration (22; 122) selon la revendication 10,
**caractérisé en ce qu'**un premier tube d'expiration (52; 152) enfermant le canal de gaz respiratoire amont (54; 154), un deuxième tube d'expiration (76; 176) enfermant le canal de gaz respiratoire aval (58; 158) et la cloison (76; 176) séparant la chambre de dérivation (74; 174) du canal de gaz respiratoire aval (58; 158) sont formés d'une seule pièce en tant que composant de canal (50; 150).

12. Agencement de soupape d'expiration (22; 122) selon l'une des revendications précédentes,
**caractérisé en ce que** le corps de soupape (64; 164) est un corps de soupape à diaphragme (64; 164) s'étendant sur une extrémité longitudinale du canal de gaz respiratoire amont (54; 154) et **en ce que** le siège de soupape (66; 166) est formé à l'extrémité longitudinale du canal de gaz respiratoire amont (54; 154).

13. Agencement de soupape d'expiration (22; 122) selon la revendication 12, en incluant la revendication 3,
**caractérisé en ce que** le corps de soupape à diaphragme (64; 164) et le diaphragme de fermeture (84; 184) sont prévus sur l'agencement de soupape d'expiration (22; 122) avec des surfaces de diaphragme mutuellement parallèles.

14. Agencement de soupape d'expiration (22; 122) selon la revendication 13,
**caractérisé en ce que** le diaphragme de fermeture (84; 184) est disposé orthogonalement à la direction d'action de la gravité (g) lorsque l'agencement de soupape d'expiration (22; 122) est disposé prêt à l'opération, son côté extérieur opposé à la chambre de dérivation (74; 174) étant orienté à l'opposé de la direction d'action de la gravité (g).

15. Dispositif respiratoire (10) pour la respiration artificielle de patients, comprenant un dispositif d'alimentation en gaz respiratoire (15), à partir duquel un conduit d'inspiration mène à une interface patient-respirateur (31), à partir de laquelle à son tour un conduit d'expiration mène à un puits de gaz respiratoire (U), tel que l'atmosphère ambiante (U),
**aractérisé en ce que** dans le conduit d'expiration un agencement de soupape d'expiration (22; 122) selon l'une des revendications précédentes est prévu dans le conduit d'expiration, dans lequel le canal de gaz respiratoire amont (54; 154) est relié par une section (42) du conduit d'expiration à l'interface respiratoire de patient (31) pour le transfert du gaz respiratoire expiratoire de l'interface respiratoire de patient (31).
